(19) Europäisches Patentamt
European Patent Office
Office européen des brevets

(11) EP 1 119 256 B1

(12) **EUROPEAN PATENT SPECIFICATION**

(45) Date of publication and mention of the grant of the patent:
**04.06.2003 Bulletin 2003/23**

(21) Application number: **99947481.0**

(22) Date of filing: **07.10.1999**

(51) Int Cl.[7]: **A01N 47/34**
// A01N47:34, A01N43:40

(86) International application number:
**PCT/EP99/07538**

(87) International publication number:
**WO 00/021371 (20.04.2000 Gazette 2000/16)**

(54) **ORAL COMBINATION OF LUFENURON AND NITENPYRAM AGAINST FLEAS**
ORALE KOMBINATION VON LUFENURON UND NITENPYRAM GEGEN FLÖHE
COMBINAISON DE LUFENURON ET DE NITENPYRAM CONTRE LES PUCES

(84) Designated Contracting States:
**AT BE CH CY DE DK ES FI FR GB GR IE IT LI LU MC NL PT SE**

(30) Priority: **09.10.1998 CH 204198**

(43) Date of publication of application:
**01.08.2001 Bulletin 2001/31**

(73) Proprietors:
- **Novartis AG**
  **4056 Basel (CH)**
  Designated Contracting States:
  **BE CH DE DK ES FI FR GB GR IE IT LI LU MC NL PT SE CY**
- **Novartis Pharma GmbH**
  **1230 Wien (AT)**
  Designated Contracting States:
  **AT**

(72) Inventors:
- **TINEMBART, Olivier**
  **CH-2800 Delémont (CH)**
- **FRANC, Michel**
  **F-31100 Toulouse (FR)**
- **STEFFAN, Jean**
  **F-68200 Mulhouse (FR)**

(74) Representative: **Grubb, Philip William et al**
**Novartis AG,**
**Corporate Intellectual Property,**
**Lichtstrasse 35**
**4002 Basel (CH)**

(56) References cited:
**WO-A-93/24002**
**WO-A-95/33380**
**WO-A-98/25466**
**WO-A-99/18796**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 119 256 B1

## Description

The present invention relates to veterinary preparations based on a combination of the nitroenamine derivatives of formula (I) named in the following with benzoylurea derivatives of formula (II) similarly named in the following, and their usage in the systemic control of fleas on domestic animals, as well as the production and usage of such preparations and combinations.

The present invention is thus concerned with a veterinary composition for the control of fleas comprising an amount that is effective against fleas of a combination of a compound of formula (I)

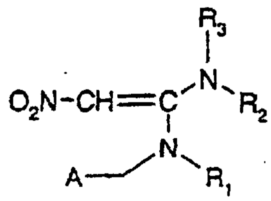

(I)

wherein

$R_1$ is hydrogen, $C_1$-$C_6$alkyl or $C_3$-$C_7$-cycloalkyl;
$R_2$ is hydrogen, $C_1$-$C_6$alkyl or $C_3$-$C_7$-cycloalkyl;
$R_3$ is hydrogen or $C_1$-$C_6$alkyl; and
A is heterocyclyl which is unsubstituted or substituted once or repeatedly by identical or different halogen atoms;

with a compound of formula (II)

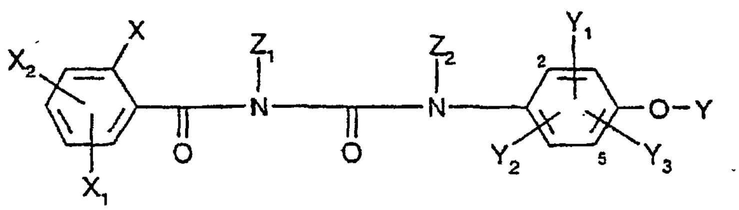

(II)

wherein

X is halogen;
$X_1$ is hydrogen or halogen;
$X_2$ is hydrogen or halogen;
$Y_1$ is hydrogen or halogen;
$Y_2$ is hydrogen or halogen;
$Y_3$ is hydrogen or halogen;
$Z_1$ is hydrogen or $C_1$-$C_3$-alkyl; and
$Z_2$ is hydrogen or $C_1$-$C_3$-alkyl

and a physiologically acceptable formulation excipient

The nitroenamine derivatives of formula (I) and the benzoylurea derivatives of formula (II) are known insecticides.

A prominent representative of the nitroenamines of formula (I) is nitenpyram of formula (III)

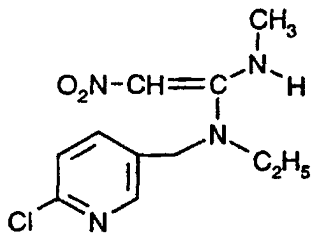

(III)

IUPAC name: (E)-N-(6-chloro-pyridin-3-ylmethyl)-N-ethyl-N'-methyl-2-nitrovinylidenediamine Nitenpyram and its preparation are described in EP-0.302.389 as example 41 on page 63. This published application discloses a rather large group of nitroenamines, of which nitenpyram is only one selected representative. These nitroenamines are described therein as contact insecticides and contact acaricides. Further nitroenamines are disclosed in EP-0.302.833. From EP-0.616.494, it is known that nitenpyram may also be administered orally to host animals. The synergistic combination of lufenuron with a number of other pesticides, including also nitenpyram, is proposed in WO 95/33380. However, the combinations proposed therein are plant pesticidal compositions for the surface treatment of plant leaves against pest infestation.

A huge number of benzoylureas, which come under formula (II), and also their preparation and usage, are described in US-5.420.163 and in the literature cited therein.

Compounds of formula (II), wherein X is F, $X_1$ is 6-F; $X_2$ is H; Y is $CF_2CHFCF_3$; $Y_1$ is 2-F; $Y_2$ is 3-Cl, $Y_3$ is 5-Cl; $Z_1$ is H, methyl or ethyl; and $Z_2$ is H, methyl or ethyl, and wherein at least $Z_1$ or $Z_2$ is methyl or ethyl, are described in WO 98/19542.

Compounds of formula (II), wherein X is F, $X_1$ is 6-F; $X_2$ is H; Y is $CF_2CHFCF_3$; $Y_1$ is 2-F; $Y_2$ is 3-Cl, $Y_3$ is 5-Cl; $Z_1$ is H, methyl or ethyl; and $Z_2$ is H, methyl or ethyl, and wherein at least $Z_1$ or $Z_2$ is methyl or ethyl, are described in WO 98/19542.

Compounds of formula (II), wherein X is F, $X_1$ is 6-F; $X_2$ is H; Y is $CF_2CHFCF_3$; $Y_1$ is 3-Cl; $Y_2$ is H, $Y_3$ is 5-Cl; $Z_1$ is H, methyl or ethyl; and $Z_2$ is H, methyl or ethyl, and wherein at least $Z_1$ or $Z_2$ is methyl or ethyl, are described in WO 98/19543.

Compounds of formula (II), wherein X is F, $X_1$ is 6-F; $X_2$ is H; Y is $CH(CH_3)CF_2R$; R is $CF_3$ or $CF_2CF_3$; $Y_1$ is 2-H or F; $Y_2$ is 3-Cl, $Y_3$ is 5-Cl; $Z_1$ is H; and $Z_2$ is H, are described in WO 98/19995.

Compounds of formula (II), wherein X is F, $X_1$ is 6-F; $X_2$ is H; Y is $CF{=}CFCF_3$ or $CF_2CF_2{=}CFCF_3$; $Y_1$ is 3-Cl; $Y_2$ is H, $Y_3$ is 5-Cl; $Z_1$ is H; and $Z_2$ is H; are described in WO 98/19994.

Combinations of a compound of formula (II), wherein X is F, $X_1$ is 6-F; $X_2$ is H; Y is $CF_2CFHOCF_3$; $Y_1$ is 3-Cl; $Y_2$ is H, $Y_3$ is H; $Z_1$ is H; and $Z_2$ is H; with other antiparasitic agents are described in WO 98/25466.

One known representative of formula (II) is lufenuron from EP-0.179.021. The substance in question here is (*R,S*)-1-[2,5-dichloro-4-(1,1,2,2,3,3,3-hexafluoropropoxy)-phenyl]-3-(2,6-difluorobenzoyl)urea.

Another known representative of formula (II) is novaluron from EP-0.271.923. The substance in question here is (±)-1-[3-chloro-4-(1,1,2,trifluoro-2-trifluormethoxyethoxy)-phenyl]-3-(2,6-difluorobenzoyl)urea.

The alkyl groups present in the definitions of the substituents may be straight-chained or branched, depending on the number of carbon atoms, and they may be for example methyl, ethyl, propyl, butyl, pentyl or hexyl, as well as the branched isomers thereof, for example isopropyl, isobutyl, sec.-butyl, tert-butyl, isopentyl, neopentyl or isohexyl. $C_3$-$C_7$-cycloalkyl is cyclopropyl, cyclobutyl, cyclopentyl, cyclohexyl or cycloheptyl. Typical radicals Y, which denote partially or completely halogenated $C_1$-$C_6$-alkyl, or completely halogenated $C_1$-$C_6$-alkyl which is interrupted by one oxygen atom, or partially or completely halogenated $C_2$-$C_6$-alkenyl, are: straight-chained or branched $C_1$-$C_6$-alkyl radicals which are partially or completely substituted by identical or different halogen atoms and whose carbon chain is not interrupted or is interrupted at one point by an oxygen atom, or straight-chained or branched $C_2$-$C_6$-alkenyl radicals with a carbon double bond, such as $OCF_3$, $OC_2F_5$, $OC_3F_7$, $OC_4F_9$, $OC_5F_{11}$, $OC_6F_{13}$, $OCF(CF_3)_2$, $OCF(C_2F_5)(CF_3)$, $OCF(C_2F_5)(C_2F_5)$, $OCF_2OCF_3$, $OCF_2OCF(C_2F_5)_2$, $OCF_2CHFOF_3$, $OCH(CF_3)CF_2CF_3$, $OCH(CF_3)CF_2C_2F_5$, $OCF{=}CFCF_3$, $OCF_2CF_2{=}CFCF_3$, $OCF_2(CF_3)CF_2{=}CFCF_3$, $OCF_2CCl_3$, $OCF_2CHCl_2$, $OCF_2CHF_2$, $OCF_2CFCl_2$, $OCF_2CHBr_2$, $OCF_2CHClF$, $OCH_2CHBrCH_2Br$, $OCF_2CHBrF$, OCClFCHClF, etc. Alkoxy radicals stem from the said alkyl groups. Halo denotes halogen and normally signifies fluorine, chlorine, bromine or iodine, preferably fluorine or chlorine, especially chlorine, whereby a partially or completely halogenated substituent may contain one or more identical or different halogen atoms. Whilst giving due consideration to the number of carbon atoms contained from case to case in the corresponding group, alkenyl is either straight-chained, for example vinyl, 1-methylvinyl, allyl, 1-butenyl or 2-hexenyl, or branched, for example isopropenyl.

In the context of the present invention, heterocyclyl is understood to mean aliphatic or aromatic cyclic radicals, which contain at least one oxygen, sulphur or nitrogen atom. Five- and six-membered heterocycles are preferred. Heterocyclyl

typically includes substituents such as dioxolanyl, pyrrolidinyl, piperidinyl, morpholinyl, pyridyl, pyrrolyl, pyrryl, furyl, thienyl, imidazolyl, tetrahydrofuryl, tetrahydropyranyl, dihydrofuryl, dihydropyranyl, isoxazolyl, oxazolyl, thiazolyl, oxazolinyl, oxazolidinyl, imidazolinyl, imidazolidinyl and dioxanyl. Preference is given especially to those which are unsubstituted or have one or two halogen atoms, halogen in this case denoting fluorine, chlorine or bromine, but especially chlorine. Of these heterocyclic radicals, pyridyl, thiazolyl and tetrahydrofuryl are especially notable. Especially preferred, however, are 6,5-dichloropyridin-3-yl, especially 6-chloropyridin-3-yl, but also 5-chlorothiazol-3-yl and tetrahydrofur-3-yl, in particular 6-chloropyridin-3-yl.
The specifically named radicals are to be regarded in the context of the present invention as the preferred embodiments. By looking into the kinetics of both classes of substance, those of the nitroenamine derivatives of formula (I) and those of the benzoylurea derivatives of formula (II), it is established that, after application to an animal, benzoylureas stand out because of their slow increase in blood counts and high blood level over longer periods of time. This means that although the activity increases very slowly, it remains at a high level for a long time. In general, the benzoylurea derivatives exhibit their full activity only days or weeks after administration. However, this effect continues for weeks or even several months. In contrast, nitroenamines exhibit their full activity almost directly after application, but it only lasts for a few hours or at most 1-2 days.

Although both classes of substance are insecticides they display a completely different mode of action. Both classes of substance do in fact break up the life cycle of the insect, but each class of substance in its own specific way. While the nitroenamine derivatives are classed as so-called adulticides which act as contact poisons, the benzoylurea derivatives belong to the class of ovicides or larvicides, depending on the rate of concentration. This means that nitroenamine derivatives as contact poisons primarily kill the adult insects living on the host animal, whereas the benzoylurea derivatives leave the adult fleas alone, but prevent oviposition or lead to the laying of sterile eggs or block the transition from one stage to the next juvenile stage.

Nitroenamines of formula (I) are extremely effective if they are administered as contact insecticides, e.g. externally, i.e. topically to the pelt of an infested host animal. They also display a certain systemic activity, i.e. if they are applied orally, parenteralty or by injection to the infested host animal. From EP-0.616.494, it is known for example that nitenpyram shows a first, substantial activity after only 30 minutes in the *in vitro* test, i.e. after feeding contaminated blood to fleas. 100% activity is attained after 24 hours. It is also described therein that, 6 hours after oral administration to cats, nitenpyram reaches an activity of 60% against the fleas present on the cats, and on the second day after renewed infestation it still gives 55% activity. From this, it may be deduced that in order to achieve complete activity, nitenpyram would have to be administered orally at one or two day intervals, which is hardly reasonable for the keeper.

The infestation of fleas on domestic animals still represents for the veterinarian and for the keeper a problem that has not yet been satisfactorily resolved.

Fleas have a very complex life cycle. This is also the reason why none of the known methods is totally satisfactory in the control thereof. The known methods either aim on the one hand to control the adult fleas in the pelt of the host animal and completely leave alone the different juvenile stages of fleas, which exist not only in the pelt of the animal, but also on the floor, in carpets, in the animal's bedding, on chairs, in the garden and all the other places with which the infested animal comes into contact, or they are intended only to control the juvenile stages.

Adult cat and dog fleas *(Ctenocephalides fells* and *C. canis)* live as blood-sucking parasites normally in the pelt of the host cat or host dog. They feed on the blood of the host animal and lay their eggs in its pelt. However, since these eggs are not self-adherent, they generally fall off quickly and may be found on the floor, in carpets, in the dog or cat basket, on chairs used by the animal, in the garden, in the backyard and therefore everywhere that the infested animal tends to go.

This means that the entire living area of the host animals is infested with flea eggs from which larvae form under normal conditions within two days. Three stages of development of the larvae may be distinguished, and each lasts three days. In the last stage, the larva spins its cocoon and is transformed into the pupa. Under favourable conditions, i.e. 33°C and at a relative humidity of 65 %, the transformation from the egg to pupa takes place in about 8 to 10 days. After about a further 8 days, the young fleas develop in the cocoons that are still on the floor, in the carpets, the bedding, the chairs, etc. The young adult fleas remain there until they sense the presence of an acceptable host animal, then they hatch from their cocoon and try to jump onto the host animal. It can be seen from this that it takes at least three weeks for a young flea that has developed from an egg to be in a position to reinfest the host animal.

However, depending on the environmental conditions, this young flea can also remain in its cocoon for months, possibly up to a year. On the other hand, under less optimum conditions, the development from egg to adult young flea may take 4 to 5 months. To reach their sexual maturity, fleas require blood as the nutrient in order to be able to reproduce, and moreover, this blood must come from the correct host animal.

This long life cycle, which passes through various stages, and may also take place in part independently of the host animal, requires special flea control compositions and methods which, up until now, have not yet been produced.

Even if the fleas in the pelt of the host animal can be successfully controlled, i.e. if all the adult fleas are killed

by an appropriate contact poison, the cat or dog is still at risk, for weeks or even months, of constant reinfestation by newly hatched young fleas from the animal's environment. A vicious circle sets in, leading to constant reinfestation, even if the host animal does not come into contact with an infested animal of the same species. The keeper of the animal often cannot see the end of this process, since he merely discovers that his apparently successfully treated animal has fleas again after a little time. This frequently leads to dissatisfaction and withdrawal of the apparently ineffective treatment.

Flea infestation of dogs and cats has unpleasant side effects not only for the animal to be treated, but also for its keeper. Flea bites lead e.g. to local skin irritation or annoying itching and often turn into severe scratching and injury of the skin, whereby severe infection consequently sets in. A large number of the frequently bitten animals become allergic in time to the flea excretions, leading to very itchy and crusty skin changes around the sites of the bites on the animal's body. These skin changes normally have a diameter of about 3 mm or more and often make the animal vicious and cause it to scratch, so that there is consequential partial hair loss. In addition, flea-infested animals are continuously exposed to the danger that they may become infected with *Dipylidium,* a kind of tapeworm that is transmitted by infected fleas and can only be controlled with great difficulty.

Flea infestation is not only extremely annoying for the infested animal, but also has unpleasant consequences for the keeper, since he finally recognises that his pet is behaving unusually and must be ill and suffering, and that he must help it. In addition, there may be unpleasant consequences for the keeper if he does away with the animal or it dies or is temporarily removed from the habitual environment, since newly hatched fleas in the floor may even be forced to attack humans if a suitable host animal has not been available for a long time, although they cannot reproduce if human blood is the only source of food. Even if the dog or cat is present, the keeper may also be bitten by the fleas.

Furthermore, dog and cat fleas, or their excretions, may lead to allergy-like skin problems of many humans, which in many cases compels the owner to do away with the pet. It has therefore always been desirable to effectively control fleas of dogs and cats.

A series of conventional control methods is known, but they have different types of disadvantages. If the keeper uses flea combs for example, then the only option is to comb the animal thoroughly and often, which can be very time-consuming depending on the size of the animal, and is not accepted patiently by every animal. Also, not every keeper is prepared or in a position to give up the time needed to do this. In certain cases, appropriate flea-active shampoos cannot be used successfully, since in particular cats and numerous dogs do not let the keepers bathe them at all or only with force. In addition, the effect of such bathing treatment lasts at most ca. one week, and the whole troublesome procedure has to be repeated. The same or quite similar problems arise when using dips or rinses. The use of dusting powders is also generally not accepted without resistance by the animal, since it takes a few minutes to treat the whole skin surface evenly, and inevitably dust reaches the mouth, nose and eyes. Even during careful application, the possibility that the powder will be inhaled by the animal and the human cannot be excluded. It is practically unavoidable that the human will also come into more or less intensive contact with the composition.

With the use of sprays, for many there will be an unpleasant surprise, since most animals, especially cats, take flight or react aggressively as soon as they hear the spraying noise. Moreover, sprays also have all the disadvantages mentioned for powders, and in addition, they are more finely dispersed in the atmosphere and therefore inhaled by humans and animals. Fleas are also controlled frequently with so-called flea collars, which guarantee good effectiveness for a transient period. A certain weakness of this treatment is, in particular, the locally very restricted application. The killing activity in the neck and chest areas is generally 100 %; however, parts of the body further away were hardly affected, In addition, there is a time limit to the activity of these collars. Besides this, many of these collars are unattractive and may disturb the animal. One can also buy discs today, which hang from customary collars and should be active. These are of an attractive appearance, but their activity is unsatisfactory because the skin contact is insufficient. A few flea-active organophosphorus compounds are available also as spot-on formulations and are thus applied to a locally limited place on the pelt. In general, they show good short-term activity against adult fleas, but the composition used often has problematic toxicity values. Organophosphorus compounds were in part also administered orally, but there are strict safety limits on these and under no circumstances may they be applied at the same time as other organophosphorus compounds.

Overall, it can be said that many of the past processes aimed at killing the adult fleas and in part gave good short-term control. However, what was previously not appreciated sufficiently was the fact that, because of the special life cycle of the fleas, dogs and cats are always reinfested, on the one hand since contact with the flea eggs, flea larvae and young adult fleas on the floor or in the closest environment of the animal is unavoidable, and on the other hand because many domestic animals come into contact over and over again with infected animals of the same species.

When the necessity to prevent constant reinfestation was recognised, special compositions were developed, which disinfected the sleeping areas of dogs and cats. Disinfecting is however only useful if the host animal itself is treated at the same time.

Furthermore, using certain benzoylureas, such as lufenuron, a class of substances was prepared which acted specifically on the juvenile stages of fleas. Their usage interrupts the life cycle of fleas very effectively, but not directly

at the start of treatment.

All in all, flea infestation of domestic animals is still an unsatisfactorily resolved problem. It is the described, very complex life cycle of the fleas, which passes through different juvenile stages, living not only on the host animal but also sometimes also in its surroundings, that makes control so much more difficult. This is also the reason why none of the known methods of control have proved satisfactory in the long term. Comprehensive flea treatment nowadays still stipulates the parallel, time- and labour-intensive usage of several methods over long periods of time. Success usually depends on treating not only the infested animal, e.g. the dog or cat, but additionally disinfecting all the locations which the infested animal frequents. The keeper of the animal not only has the work, but also has to take into consideration the surroundings which are contaminated with the active substances.

Until now, if domestic animals were to be given comprehensive and long-lasting protection against both the juvenile flea stages and the adults, several separate treatment methods had to be carried out. To eliminate adults on the host animal, rapidly acting contact adulticides were administered usually topically at short intervals, while the juvenile stages were controlled usually orally with long-term preparations. Elimination of all stages living in the surroundings of the host animal was effected by repeatedly disinfecting all the places that the host animal frequented. Both veterinarians and keepers have long wished for a simple but effective process.

The different course of efficacy and in particular the very different duration of efficacy of adulticides, such as nitroenamines, compared with ovicides or larvicides, such as benzoylureas, has so far deterred the person skilled in the art from using a combination of both classes of substance.

Besides this, numerous unsuccessful attempts were also made to eliminate the short duration of efficacy, which was rather unsatisfactory for practical application, in the systemic administration of nitroenamines. It was shown that a prolonging of the systemic action by raising the concentration of administration can only be achieved up to a point, because the active substance is excreted much too quickly. On the other hand, if one wanted to insert depot forms under the skin or in the muscles, which release the active ingredient over several weeks, the amounts that would have to be injected or implanted would be so large that they would no longer be acceptable to the host animal, as they lead to substantial irritation. Therefore this solution breaks down for ethical and practical reasons. It was similarly established that long-term efficacy by means of increased oral administration is also not attainable, because a larger amount administered does not in fact have an effect on the duration of efficacy. When administered orally, e.g. as tablets, nitroenamines are absorbed and dispersed very rapidly in the blood via the gastro-intestinal tract, but are excreted just as rapidly via the urine. The half-life in the blood of ca. 7-9 hours for cats and ca. 3-5 hours for dogs is much too short to achieve an improvement in efficacy by means of raising the dose. A very steep rise in blood values after application is observed, and an equally very rapid flattening of the curve, without a significant influence on prolonged bioavailability. This has serious effects on systemically employable preparations, e.g. tablets or injections. Because of the short duration of efficacy, tablets or injections have to be administered at short intervals, preferably every other day, which means that the keeper has to repeat the treatment or visit the vet too frequently. An intensive treatment concept of this kind requires a great amount of discipline and experience has shown that this causes stress to the animal and the keeper after only a short time. This often manifests itself as an aversion to the treatment and leads to discontinuation of the treatment. Therefore, a prolonging of the systemic action of this extremely active class of substances was an aim that had been desired for a long time, but apparently not achieved.

Although the nitroenamine derivatives of formula (I) exhibit acceptable anti-flea activity only for a few hours up to a maximum of 2 days when administered orally to a host animal, we have now surprisingly succeeded in prolonging their duration of efficacy to at least two weeks by combining them with benzoylurea derivatives of formula (II). It would be expected that when administering lufenuron, the nitenpyram would have to be administered over the entire treatment period at weekly intervals in order to maintain the full broad-band effect against all stages of development. However, this is not the case at all. It has surprisingly been established that a six-week treatment of nitenpyram suffices completely. It was totally unexpected that no reinfestation of any kind would occur afterwards. This is even more surprising, since the nitenpyram may even be given at an interval of two weeks.

In short, it was found that by means of systemic administration, e.g. orally, parenterally or by implant, of an amount of the combination according to the invention, which is effective against fleas, of a compound of formula (I) and a compound of formula (II), the flea infestation of domestic animals can be completely prevented from the second week of treatment and over the whole duration of treatment, and even the environment of the host animal remains free of fleas for a long time. This does not happen either during treatment with nitenpyram alone or with lufenuron alone.

This desired effect is thereby attained not only after injection or with an implant, but also even after oral administration, that is via the roundabout route through the gastro-intestinal tract and thus through the contaminated blood.

Both the compounds of formula (I) and the compounds of formula (II) are notable for their excellent anti-flea activity, and by combining (I) and (II), not only are adult fleas quickly killed, but also all the juvenile flea stages. Flea larvae that hatch from the flea eggs basically feed on the excretions of the adult fleas. Since the combinations according to the invention similarly quickly kill the adult fleas, the necessary excretions which form the basis of food for the juvenile

stages are missing. This basis of food is now absent, so that the juvenile stages are destroyed before they reach the adult stage. If, in fact, a few young fleas develop, they are eliminated by the contact action.

The present invention thus relates to a veterinary composition for a systemic, and thus problem-free, and in particular comprehensive, long-term control of fleas, which sets in immediately after treatment and continues for a long period of time.

What is essential to the invention is that the combinations according to the invention are administered systemically. Oral application is preferred, however, especially the administration of tablets or suspensions. Excellent activity is also obtained with other different types of application, e.g. by injecting the formulated compounds or mixing with food. In the context of the present invention, formulated means e.g. in the form of a powder, a tablet, a granulate, a capsule, an emulsion, a suspension, a foam, in micro-encapsulated form, etc., whereby as already mentioned, the preparation does not necessarily have to be given to the animal directly, but may also be conveniently mixed with its food. Of course, all compositions to be administered orally may contain further additives, in addition to conventional formulation excipients. These additives encourage willing consumption by the host animal, for example suitable odorous substances, flavourings and/or taste substances.

Because of its simple practicability, oral usage is one of the preferred subjects of the invention. A further type of application is parenteral usage, e.g. by subcutaneous injection or injection into the vein or the long-term preparation (depot form) in the form of an implant.

Oral application also includes e.g. administration of dog and cat food, which contains the combination according to the invention of compounds of formula (I) and (II) already mixed therein, e.g. as biscuits or as titbits, as chews, as water-soluble capsules or tablets, in water-soluble form that can be dripped onto the food, or in other forms that can be mixed with the animal food. The implants also include all the devices which can be inserted into the body of the animal in order to deliver the substance, e.g. so-called mini-pumps.

Percutaneous application forms include for example the subcutaneous, dermal, intramuscular and even intravenous administration of injectable forms. Apart from the usual injection syringes with needles, needleless gun-type apparatus may also be used.

By choosing an appropriate formulation, it is possible to enhance the penetration power of a combination according to the invention through the living tissue of the animal, or to maintain its availability. This is of importance e. g. if a less soluble composition is used, the low solubility of which requires a solubility-enhancing measure, since the body fluids of the animal are only able to dissolve small amounts of the substance at a time.

Furthermore, a combination according to the invention may also be present in a matrix formulation in order to achieve a greatly delayed release of active ingredient, this matrix formulation physically preventing its release and premature secretion, and maintaining the bioavailability of the active ingredient. This matrix formulation is injected into the body e.g. intramuscularly or subcutaneously and remains there as a type of depot, from which the active ingredients are continuously released. Such matrix formulations are known to the person skilled in the art. These are generally waxy, semi-solid substances, for example plant waxes and polyethylene glycols with a high molecular weight. Both active ingredient compositions can be used either in the same matrix or in different matrices, which are used in different parts of the body if required.

Long-term availability of the active ingredient combination is also achieved by inserting an implant of the active substances into the animal. Such implants are widely used in veterinary medicine and often consist of silicone-containing rubber. Here, the active substances are dispersed in the solid rubber or are found in the inside of a hollow rubber element. Care must be taken that a combination according to the invention is selected, which is soluble in the rubber implant, since it is first dissolved in the rubber and then continuously seeps from the rubber material to the body fluids of the animal to be treated. Both classes of active ingredient can be used either in the same implant or in different implants and in various parts of the body.

The rate of release of the active substances from the implant, and thus the time span during which the implant shows activity, is generally determined by the accuracy of measurement (amount of active ingredient in the implant) of the implant, the environment of the implant and the polymer formulation from which the implant is made.

The administration of a combination according to the invention by means of an implant represents a further preferred constituent of the present invention. This type of administration is economical and effective, because a correctly dimensioned implant guarantees a constant concentration of the active substance in the tissue of the host animal. Nowadays, implants can be designed and implanted in a simple manner, so that they are in a position to deliver the active ingredient over some months. After inserting the implant, there is no need to trouble the animal again, and there is also no longer any need to worry about the dosage.

The administration of veterinary medicine additives to animal food is best known in the field of animal health. Usually, first of all, a so-called premix is produced, in which the active substances are dispersed in a liquid or finely distributed in solid carriers. This premix may normally contain, depending on the desired final concentration in the food, about 1 to X g of a nitroenamine of formula (I) and about 1 to Y g of a benzoylurea of formula (II) per kg of premix, whereby X and Y are values between 10 and 15 and depend on the body weight of the host animal.

Since the combinations according to the invention can be affected by constituents in the food, they should preferably be formulated in a protective matrix, e.g. in gelatin, before adding to the premix.

The present invention thus also relates to the aspect of a veterinary composition for flea control which is characterised by an active ingredient combination according to the invention which is administered to the host animal with the food.

A combination according to the invention is conveniently taken in the following dosages:

Nitroenamine of formula (I) from 0.01 to 800 mg/kg, preferably 0.1 to 200 mg/kg, especially 0.5 to 30 mg/kg body weight, based on the host animal;
Benzoylurea of formula (II) from 0.01 to 800 mg/kg, preferably 0.1 to 200 mg/kg, especially 0.5 to 30 mg/kg body weight, based on the host animal; whereby oral administration is preferred over all the others.

A good dosage that can be administered regularly to the host animal is 0.5 to 100 mg/kg body weight of a nitroenamine of formula (I) to be given twice or preferably once each week, and the parallel administration of 0.5 to 100 mg/kg body weight of a benzoylurea of formula (II) given monthly to three-monthly. The administration interval for the benzoylurea of formula (II) may even be extended at a later treatment phase to half a year, whereby the addition of the nitroenamine of formula (I) may even be dispensed with if there is little infestation from the environment of the domestic animal.

The arrangement under which a combination preparation of a nitroenamine of formula (I) and a benzoylurea of formula (II) is administered may be carried out in many different ways. The user, whether the veterinarian giving the treatment or the keeper, may have at his disposal specially designed packs, in which either separate unit doses are present for each active ingredient, e.g. tablets which contain a specific single dose of the active ingredient and have to be applied according to a certain administration scheme, or in which specially characterised unit doses are present, e.g. tablets which contain both active ingredients in the same tablet and in the relevant concentrations, and have to be administered at given intervals.

The total dose for the same active ingredient may vary from one species of animal to another and even within one species, since it depends *inter alia* on the weight, the age and the constitution of the host animal.

With the combinations according to the invention, the active ingredients are normally not applied in pure form, but preferably in the form of a composition or preparation which contains, in addition to the active ingredient, application-enhancing constituents or formulation excipients, whereby such constituents are beneficial to the host animal.

Such compositions or preparations to be used according to the invention usually contain 0.1 to 99 % by weight, especially 0.1 to 95 % by weight, of a nitroenamine of formula (I) or of a benzoylurea of formula (II) or of both active ingredients, and 99.9 to 1 % by weight, especially 99.9 to 5 % by weight, of a solid or liquid, physiologically acceptable carrier, including 0 to 25 % by weight, especially 0.1 to 25 % by weight, or a non-toxic surfactant.

Whereas it is preferred to formulate commercial products as concentrates, the end user will normally use dilute formulations.

Such compositions may also contain further additives, such as stabilisers, anti-foaming agents, viscosity regulators, binding agents or tackifiers, as well as other active ingredients, in order to achieve special effects.

The formulation excipients which may be used are the physiologically acceptable carriers which are known from veterinary medicine for oral and parenteral administration and for administration by implants. A few examples are mentioned hereinafter.

Suitable carriers are in particular fillers, such as sugars, e.g. lactose, saccharose, mannitol or sorbitol, cellulose preparations and/or calcium phosphates, e.g. tricalcium phosphate or calcium hydrogen phosphate, in a broader sense also binders, such as starch pastes using e.g. corn, wheat, rice or potato starch, gelatin, tragacanth, methyl cellulose and/or, if desired, disintegrants, such as the above-mentioned starches, in a broader sense also carboxymethyl starch, cross-linked polyvinylpyrrolidone, agar, alginic acid or a salt thereof, such as sodium alginate. Excipients are especially flow conditioners and lubricants, for example silicic acid, talc, stearic acid or salts thereof, such as magnesium or calcium stearate, and/or polyethylene glycol. Tablet cores may be provided with suitable, where appropriate, gastric-juice-resistant coatings, using *inter alia* concentrated sugar solutions which may comprise gum arabic, talc, polyvinylpyrrolidone, polyethylene glycol and/or titanium dioxide, or coating solutions in suitable organic solvents or solvent mixtures, or, for the preparation of gastric-juice-resistant coatings, solutions of suitable cellulose preparations, such as acetylcellulose phthalate or hydroxypropylmethylcellulose phthalate. Dyes, flavours or pigments may be added to the tablets or tablet coatings, for example for identification purposes or to indicate different doses of active ingredient.

Further orally administrable preparations are hard capsules consisting of gelatin, and also soft, sealed capsules consisting of gelatin and a plasticizer, such as glycerol or sorbitol. The hard capsules may contain the active ingredient in the form of granules, for example in admixture with fillers, such as lactose, binders, such as starches, and/or glidants, such as talc or magnesium stearate, and where appropriate stabilizers. In soft capsules, the active

ingredients are preferably dissolved or suspended in suitable liquids, such as fatty oils, paraffin oil, or liquid polyethylene glycols, and stabilizers may likewise be added. Amongst other forms, capsules which can be both easily chewed and also swallowed whole are preferred.

The formulations suitable for parenteral administration are especially aqueous solutions of the active ingredients in water-soluble form, e.g. a water-soluble salt, in the broader sense also suspensions of the active ingredients, such as appropriate oily injectable suspensions using suitable lipophilic solvents or vehicles, such as oils, e.g. sesame oil, or synthetic fatty acid esters, e.g. ethyl oleate, or triglycerides, or aqueous injectable suspensions containing viscosity-increasing agents, e.g. sodium carboxymethyl cellulose, sorbitol and/or dextran, and where appropriate stabilizers.

The compositions of the present invention may be prepared in a manner known *per se,* for example by means of conventional mixing, granulating, coating, dissolving or lyophilising processes. Pharmaceutical compositions for oral administration can be obtained, for example, by combining the active ingredient(s) with solid carriers, granulating a resulting mixture where appropriate, and processing the mixture or granules, if desired or necessary, to form tablets or tablet cores following the addition of suitable excipients.

Administration of the combinations according to the invention may take place either by transforming both active ingredients as a true mixture into a single dosage form and administering to the host animal, or by administering to the host animal in separate dosage forms, so that the active substances are only mixed together in the body fluids, especially in the blood. The latter method has the advantage that the active substances can also be applied with different time delays. For example, the benzoylurea which is effective over long periods can be applied at longer intervals, and the shorter-acting nitroenamine derivative can be applied at somewhat shorter intervals. This procedure leads to a reduction in the amount of active substance used.

It is also conceivable to have applications in which a pack is available to the keeper, in which the single dosage forms contain different amounts of benzoylurea and nitroenamine derivative, and the user follows an administration scheme.

Consequently, in the combinations according to the invention or the usage of the compounds of formula (I) and (II) according to the invention, the active ingredients in the dosage form may represent true mixtures, but they may also be administered individually and form mixtures only when they are in the host organism. What is crucial is that they are present together for certain periods at latest in the host organism, so that the desired effect arises.

Preferred compounds of formulae (I) and (II) within the context of the present invention are shown in the following Tables 1 and 2.

Table 1: Compounds of formula (I), wherein A is

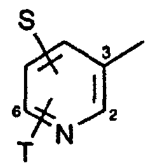

| No. | S | T | $R_1$ | $R_2$ | $R_3$ |
|---|---|---|---|---|---|
| 1.01 | 6-Cl | H | $C_2H_5$ | H | $CH_3$ |
| 1.02 | 6-Cl | 5-Cl | $C_2H_5$ | H | $CH_3$ |
| 1.03 | 6-Cl | 5-F | $C_2H_5$ | H | $CH_3$ |
| 1.04 | 6-Cl | 4-F | $C_2H_5$ | H | $CH_3$ |
| 1.05 | 6-Cl | H | $CH_3$ | H | $CH_3$ |
| 1.06 | 6-Cl | 5-Cl | $CH_3$ | H | $CH_3$ |
| 1.07 | 6-Cl | 2-F | $CH_3$ | H | $CH_3$ |
| 1.08 | 6-Cl | 4-F | $CH_3$ | H | $CH_3$ |
| 1.09 | 6-Cl | H | H | H | $CH_3$ |
| 1.10 | 6-Cl | 5-Cl | H | H | $CH_3$ |
| 1.11 | 6-Cl | 5-F | H | H | $CH_3$ |
| 1.12 | 6-Cl | 4-F | H | H | $CH_3$ |
| 1.13 | 6-Cl | H | $nC_3H_7$ | H | $CH_3$ |
| 1.14 | 6-Cl | 5-Cl | $nC_3H_7$ | H | $CH_3$ |
| 1.15 | 6-Cl | 5-F | $nC_3H_7$ | H | $CH_3$ |
| 1.16 | 6-Cl | 4-F | $nC_3H_7$ | H | $CH_3$ |
| 1.17 | 6-Cl | H | $iC_3H_7$ | H | $CH_3$ |
| 1.18 | 6-Cl | H | $C_2H_5$ | $CH_3$ | $CH_3$ |
| 1.19 | 6-Cl | 5-Cl | $C_2H_5$ | $CH_3$ | $CH_3$ |
| 1.20 | 6-Cl | 5-F | $C_2H_5$ | $CH_3$ | $CH_3$ |
| 1.21 | 6-Cl | 4-F | $C_2H_5$ | $CH_3$ | $CH_3$ |

Table 2:

| Preferred benzoylureas of formula (II) | | | | | | | | | |
|---|---|---|---|---|---|---|---|---|---|
| No. | X | $X_1$ | $X_2$ | $Z_1$ | $Z_2$ | Y | $Y_1$ | $Y_2$ | $Y_3$ |
| 2.01 | F | 6-F | H | H | H | $CF_2CHFCF_3$ | 2-Cl | 5-Cl | H |
| 2.02 | F | 6-F | H | H | H | $CF_2CHFCF_3$ | 2-F | 3-Cl | 5-Cl |
| 2.03 | F | 6-F | H | H | $CH_3$ | $CF_2CHFCF_3$ | 2-F | 3-Cl | 5-Cl |
| 2.04 | F | 6-F | H | H | $C_2H_5$ | $CF_2CHFCF_3$ | 2-F | 3-Cl | 5-Cl |
| 2.05 | F | 6-F | H | $CH_3$ | H | $CF_2CHFCF_3$ | 2-F | 3-Cl | 5-Cl |
| 2.05 | F | 6-F | H | $CH_3$ | $CH_3$ | $CF_2CHFCF_3$ | 2-F | 3-Cl | 5-Cl |
| 2.07 | F | 6-F | H | $CH_3$ | $C_2H_5$ | $CF_2CHFCF_3$ | 2-F | 3-Cl | 5-Cl |
| 2.08 | F | 6-F | H | $C_2H_5$ | H | $CF_2CHFCF_3$ | 2-F | 3-Cl | 5-Cl |
| 2.09 | F | 6-F | H | $C_2H_5$ | $CH_3$ | $CF_2CHFCF_3$ | 2-F | 3-Cl | 5-Cl |
| 2.10 | F | 6-F | H | $C_2H_5$ | $C_2H_5$ | $CF_2CHFCF_3$ | 2-F | 3-Cl | 5-Cl |

Table 2: (continued)

| Preferred benzoylureas of formula (II) | | | | | | | | | |
|---|---|---|---|---|---|---|---|---|---|
| **No.** | **X** | **$X_1$** | **$X_2$** | **$Z_1$** | **$Z_2$** | **Y** | **$Y_1$** | **$Y_2$** | **$Y_3$** |
| 2.11 | F | 6-F | H | H | H | $CF_2CHFCF_3$ | 3-Cl | H | 5-Cl |
| 2.12 | F | 6-F | H | H | $CH_3$ | $CF_2CHFCF_3$ | 3-Cl | H | 5-Cl |
| 2.13 | F | 6-F | H | H | $C_2H_5$ | $CF_2CHFCF_3$ | 3-Cl | H | 5-Cl |
| 2.14 | F | 6-F | H | $CH_3$ | H | $CF_2CHFCF_3$ | 3-Cl | H | 5-Cl |
| 2.15 | F | 6-F | H | $CH_3$ | $CH_3$ | $CF_2CHFCF_3$ | 3-Cl | H | 5-Cl |
| 2.16 | F | 6-F | H | $CH_3$ | $C_2H_5$ | $CF_2CHFCF_3$ | 3-Cl | H | 5-Cl |
| 2.17 | F | 6-F | H | $C_2H_5$ | H | $CF_2CHFCF_3$ | 3-Cl | H | 5-Cl |
| 2.18 | F | 6-F | H | $C_2H_5$ | $CH_3$ | $CF_2CHFCF_3$ | 3-Cl | H | 5-Cl |
| 2.19 | F | 6-F | H | $C_2H_5$ | $C_2H_5$ | $CF_2CHFCF_3$ | 3-Cl | H | 5-Cl |
| 2.20 | F | 6-F | H | H | H | $CH(CH_3)C_2F_5$ | 3-Cl | H | 5-Cl |
| 2.21 | F | 6-F | H | H | H | $CH(CH_3)C_2F_5$ | 3-Cl | 2-F | 5-Cl |
| 2.22 | F | 6-F | H | H | H | $CH(CH_3)C_2F_4CF_3$ | 3-Cl | H | 5-Cl |
| 2.23 | F | 6-F | H | H | H | $CH(CH_3)C_2F_4CF_3$ | 3-Cl | 2-F | 5-Cl |
| 2.24 | F | 6-F | H | H | H | $CF{=}CFCF_3$ | 3-Cl | H | 5-Cl |
| 2.25 | F | 6-F | H | H | H | $CF_2CF_2{=}CFCF_3$ | 3-Cl | H | 5-Cl |
| 2.28 | F | 6-F | H | H | H | $CF_3$ | 2-Cl | 5-Cl | H |
| 2.29 | F | 6-F | H | H | H | $CF_2CHClF$ | 2-Cl | 5-Cl | H |
| 2.30 | F | 6-F | H | H | H | $CF_2CHCHCl_2$ | 2-Cl | 5-Cl | H |
| 2.31 | F | 6-F | H | H | H | $CF_2CHCFBr$ | 2-Cl | 5-Cl | H |
| 2.32 | F | H | H | H | H | $CF_2CHFCF_3$ | 2-Cl | 5-Cl | H |
| 2.33 | Cl | H | H | H | H | $CF_2CHFCF_3$ | 2-Cl | 5-Cl | H |
| 2.34 | F | 6-Cl | H | H | H | $CF_2CHFCF_3$ | 2-Cl | 5-Cl | H |

The following combinations of compounds of formula (I) and formula (II) are preferred in particular: 1.01 & 2.01; 1.01 & 2.02; 1.01 & 2.03; 1.01 & 2.04; 1.01 & 2.05; 1.01 & 2.06; 1.01 & 2.07; 1.01 & 2.08; 1.01 & 2.09; 1.01 & 210; 1.01 & 211; 1.01 & 2.12; 1.01 & 2.13; 1.01 & 2.14; 1.01 & 2.15; 1.01 & 2.16; 1.01 & 2.17; 1.01 & 2.18; 1.01 & 2.19; 1.01 & 2.20; 1.01 & 2.21; 1.01 & 2.22; 1.01 & 2.23; 1.01 & 2.24; 1.01 & 2.25; 1.01 & 2.28; 1.01 & 2.29; 1.01 & 2.30; 1.01 & 2.31; 1.01 & 2.32; 1.01 & 2.33; 1.01 & 2.34; 1.02 & 2.01; 1.02 & 2.02; 1.02 & 2.03; 1.02 & 2.04; 1:02 & 2.05; 1.02 & 2.06; 1.02 & 2.07; 1.02 & 2.08; 1.02 & 2.09; 1.02 & 210; 1.02 & 211; 1.02 & 2.12; 1.02 & 2.13; 1.02 & 2.14; 1.02 & 2.15; 1.02 & 2.16; 1.02 & 2.17; 1.02 & 2.18; 1.02 & 2.19; 1.02 & 2.20; 1.02 &2.21; 1.02 & 2.22; 1.02 & 2.23; 1.02 & 2.24; 1.02 & 2.25; 1.02 & 2.28; 1.02 & 2.29; 1.02 & 2.30; 1.02 & 2.31; 1.02 & 2.32; 1.02 & 2.33; 1.02 & 2.34; 1.03 & 2.01; 1.03 & 2.02; 1.03 & 2.03; 1.03 & 2.04; 1.03 & 2.05; 1.03 & 2.06; 1.03 & 2.07; 1.03 & 2.08; 1.03 & 2.09; 1.03 & 210; 1.03 & 211; 1.03 & 2.12; 1.03 & 2.13; 1.03 & 2.14; 1.03 & 2.15; 1.03 & 2.16; 1.03 & 2.17; 1.03 & 2.18; 1.03 & 2.19; 1.03 & 2.20; 1.03 & 2.21; 1.03 & 2.22; 1.03 & 2.23; 1.03 & 2.24; 1.03 & 2.25; 1.03 & 2.28; 1.03 & 2.29; 1.03 & 2.30; 1.03 & 2.31; 1.03 & 2.32; 1.03 & 2.33; 1.03 & 2.34; 1.04 & 2.01; 1.04 & 2.02; 1.04 & 2.03; 1.04 & 2.04; 1.04 & 2.05; 1.04 & 2.06; 1.04 & 2.07; 1.04 & 2.08; 1.04 & 2.09; 1.04 & 210; 1.04 & 211; 1.04 & 2.12; 1.04 & 2.13; 1.04 & 2.14; 1.04 & 2.15; 1.04 & 2.16; 1.04 & 2.17; 1.04 & 2.18; 1.04 & 2.19; 1.04 & 2.20; 1.04 & 2.21; 1.04 & 2.22; 1.04 & 2.23; 1.04 & 2.24; 1.04 & 2.25; 1.04 & 2.28; 1.04 & 2.29; 1.04 & 2.30; 1.04 & 2.31; 1.04 & 2.32; 1.04 & 2.33; 1.04 & 2.34; 1.05 & 2.01; 1.05 & 2.02; 1.05 & 2.03; 1.05 & 2.04; 1.05 & 2.05; 1.05 & 2.06; 1.05 & 2.07; 1.05 & 2.08; 1.05 & 2.09; 1.05 & 210; 1.05 & 211; 1.05 & 2.12; 1.05 & 2.13; 1.05 & 2.14; 1.05 & 2.15; 1.05 & 2.16; 1.05 & 2.17; 1.05 & 2.18; 1.05 & 2.19; 1.05 & 2.20; 1.05 & 2.21; 1.05 & 2.22; 1.05 & 2.23; 1.05 & 2.24; 1.05 & 2.25; 1.05 & 2.28; 1.05 & 2.29; 1.05 & 2.30; 1.05 & 2.31; 1.05 & 2.32; 1.05 & 2.33; 1.05 & 2.34; 1.06 & 2.01; 1.06 & 2.02; 1.06 & 2.03; 1.06 & 2.04; 1.06 & 2.05; 1.06 & 2.06; 1.06 & 2.07; 1.06 & 2.08; 1.06 & 2.09; 1.06 & 210; 1.06 & 211; 1.06 & 2.12; 1.06 & 2.13; 1.06 & 2.14; 1.06 & 2.15; 1.06 & 2.16; 1.06 & 2.17; 1.06 & 2.18; 1.06 & 2.19; 1.06 & 2.20; 1.06 & 2.21; 1.06 & 2.22; 1.06 & 2.23; 1.06 &

2.24; 1.06 & 2.25; 1.06 & 2.28; 1.06 & 2.29; 1.06 & 2.30; 1.06 & 2.31; 1.06 & 2.32; 1.06 &2.33; 1.06 & 2.34; 1.07 & 2.01; 1.07 & 2.02; 1.07 & 2.03; 1.07 & 2.04; 1.07 & 2.05; 1.07 & 2.06; 1.07 & 2.07; 1.07 & 2.08; 1.07 & 2.09; 1.07 & 210; 1.07 & 211; 1.07 & 2.12; 1.07 & 2.13; 1.07 & 2.14; 1.07 & 2.15; 1.07 & 2.16; 1.07 & 2.17; 1.07 & 2.18; 1.07 & 2.19; 1.07 & 2.20; 1.07 & 2.21; 1.07 & 2.22; 1.07 & 2.23; 1.07 & 2.24; 1.07 & 2.25; 1.07 & 2.28; 1.07 & 2.29; 1.07 & 2.30; 1.07 & 2.31; 1.07 & 2.32; 1.07 & 2.33; 1.07 & 2.34; 1.08 & 2.01; 1.08 & 2.02; 1.08 & 2.03; 1.08 & 2.04; 1.08 & 2.05; 1.08 & 2.06; 1.08 & 2.07; 1.08 & 2.08; 1.08 & 2.09; 1.08 & 210; 1.08 & 211; 1.08 & 2.12; 1.08 & 2.13; 1.08 & 2.14; 1.08 & 2.15; 1.08 & 2.16; 1.08 & 2.17; 1.08 & 2.18; 1.08 & 2.19; 1.08 & 2.20; 1.08 & 2.21; 1.08 & 2.22; 1.08 & 2.23; 1.08 & 2.24; 1.08 & 2.25; 1.08 & 2.28; 1.08 & 2.29; 1.08 & 2.30; 1.08 & 2.31; 1.08 & 2.32; 1.08 & 2.33; 1.08 & 2.34; 1.09 & 2.01; 1.09 & 2.02; 1.09 & 2.03; 1.09 & 2.04; 1.09 & 2.05; 1.09 & 2.06; 1.09 & 2.07; 1.09 & 2.08; 1.09 & 2.09; 1.09 & 210; 1.09 & 211; 1.09 & 2.12; 1.09 & 2.13; 1.09 & 2.14; 1.09 & 2.15; 1.09 & 2.16; 1.09 & 2.17; 1.09 & 2.18; 1.09 & 2.19; 1.09 & 2.20; 1.09 & 2.21; 1.09 & 2.22; 1.09 & 2.23; 1.09 &2.24; 1.09 & 2.25; 1.09 & 2.28; 1.09 & 2.29; 1.09 & 2.30; 1.09 & 2.31; 1.09 & 2.32; 1.09 & 2.33; 1.09 & 2.34; 1.10 & 2.01; 1.10 & 2.02; 1.10 & 2.03; 1.10 & 2.04; 1.10 & 2.05; 1.10 & 2.06; 1.10 & 2.07; 1.10 & 2.08; 1.10 & 2.09; 1.10 & 210; 1.10 & 211; 1.10 & 2.12; 1.10 & 2.13; 1.10 & 2.14; 1.10 & 2.15; 1.10 & 2.16; 1.10 & 2.17; 1.10 & 2.18; 1.10 & 2.19; 1.10 & 2.20; 1.10 & 2.21; 1.10 & 2.22; 1.10 & 2.23; 1.10 & 2.24; 1.10 & 2.25; 1.10 & 2.28; 1.10 & 2.29; 1.10 & 2.30; 1.10 & 2.31; 1.10 & 2.32; 1.10 & 2.33; 1.10 & 2.34; 1.11 & 2.01; 1.11 & 2.02; 1.11 &2.03;1.11 & 2.04; 1.11 &2.05;1.11 & 2.06; 1.11 & 2.07; 1.11 & 2.08; 1.11 & 2.09; 1.11 & 210; 1.11 & 211; 1.11 & 2.12; 1.11 & 2.13; 1.11 & 2.14; 1.11 & 2.15; 1.11 & 2.16; 1.11 & 2.17; 1.11 & 2.18; 1.11 & 2.19; 1.11 & 2.20; 1.11 & 2.21; 1.11 & 2.22; 1.11 & 2.23; 1.11 & 2.24; 1.11 & 2.25; 1.11 & 2.28; 1.11 & 2.29; 1.11 & 2.30; 1.11 & 2.31; 1.11 & 2.32; 1.11 & 2.33; 1.11 & 2.34; 1.12 & 2.01; 1.12 & 2.02; 1.12 & 2.03; 1.12 & 2.04; 1.12 & 2.05; 1.12 & 2.06; 1.12 & 2.07; 1.12 & 2.08; 1.12 & 2.09; 1.12 & 210; 1.12 & 211; 1.12 & 2.12; 1.12 & 2.13; 1.12 & 2.14; 1.12 & 2.15; 1.12 & 2.16; 1.12 & 2.17; 1.12 & 2.18; 1.12 & 2.19; 1.12 & 2.20:1.12 & 2.21:1.12 & 2.22; 1.12 & 2.23; 1.12 & 2.24; 1.12 & 2.25; 1.12 & 2.28; 1.12 & 2.29; 1.12 & 2.30; 1.12 & 2.31; 1.12 & 2.32; 1.12 & 2.33; 1.12 & 2.34; 1.13 & 2.01; 1.13 & 2.02; 1.13 & 2.03; 1.13 & 2.04; 1.13 & 2.05; 1.13 & 2.06; 1,13 & 2.07; 1.13 & 2.08; 1.13 & 2.09; 1.13 & 210; 1.13 & 211; 1.13 & 2.12; 1.13 & 2.13; 1.13 & 2.14; 1.13 & 2.15; 1.13 & 2.16; 1.13 & 2.17; 1.13 & 2.18; 1.13 & 2.19; 1.13 & 2.20; 1.13 & 2.21; 1.13 & 2.22; 1.13 & 2.23; 1.13 & 2.24; 1.13 & 2.25; 1.13 & 2.28; 1.13 & 2.29; 1.13 & 2.30; 1.13 & 2.31; 1.13 & 2.32; 1.13 & 2.33; 1.13 & 2.34; 1.14 & 2.01; 1.14 & 2.02; 1.14 & 2.03; 1.14 & 2.04; 1.14 & 2.05; 1.14 & 2.06; 1.14 & 2.07; 1.14 & 2.08; 1.14 & 2.09; 1.14 & 210; 1.14 & 211; 1.14 & 2.12; 1.14 & 2.13; 1.14 & 2.14; 1.14 & 2.15; 1.14 & 2.16; 1.14&2.17; 1.14 & 2.18; 1.14 & 2.19; 1.14 & 2.20; 1.14 & 2.21; 1.14 & 2.22; 1.14 & 2.23; 1.14 & 2.24; 1.14 & 2.25; 1.14 & 2.28; 1.14 & 2.29; 1.14 & 2.30; 1.14 & 2.31; 1.14 & 2.32; 1.14 & 2.33; 1.14 & 2.34; 1.15 & 2.01; 1.15 & 2.02; 1.15 & 2.03; 1.15 & 2.04; 1.15 & 2.05; 1.15 & 2.06; 1.15 & 2.07; 1.15 & 2.08; 1.15 & 2.09; 1.15 & 210; 1.15 & 211; 1.15 & 2.12; 1.15 & 2.13; 1.15 & 2.14; 1.15 & 2.15; 1.15 & 2.16; 1.15 & 2.17; 1.15 & 2.18; 1.15 & 2.19; 1.15 & 2.20; 1.15 & 2.21; 1.15 & 2.22; 1.15 & 2.23; 1.15 & 2.24; 1.15 & 2.25; 1.15 & 2.28; 1.15 & 2.29; 1.15 & 2.30; 1.15 & 2.31; 1.15 & 2.32; 1.15 & 2.33; 1.15 & 2.34; 1.16 & 2.01; 1,16 & 2.02; 1.16 & 2.03; 1.16 & 2.04; 1.16 & 2.05; 1.16 & 2.06; 1.16 & 2.07; 1.16 & 2.08; 1.16 & 2.09; 1.16 & 210; 1.16 & 211; 1.16 & 2.12; 1.16 & 2.13; 1.16 & 2.14; 1.16 & 2.15; 1.16 & 2.16; 1.16 & 2.17; 1.16 & 2.18.1.16 & 2.19; 1.16 & 2.20; 1.16 & 2.21. 1.16 & 2.22.1.16 & 2.23; 1.16 & 2.24; 1.16 & 2.25; 1.16 & 2.28; 1.16 & 2.29; 1.16 & 2.30; 1.16 & 2.31; 1.16 & 2.32; 1.16 & 2.33; 1.16 & 2.34; 1.17 & 2.01; 1.17 & 2.02; 1.17 & 2.03; 1.17 & 2.04; 1.17 & 2.05; 1.17 & 2.06; 1.17 & 2.07; 1.17 & 2.08; 1.17 & 2.09; 1.17 & 210; 1.17 & 211; 1.17 & 2.12; 1.17 & 2.13; 1.17 & 2.14; 1.17 & 2.15; 1.17 & 2.16; 1.17 & 2.17; 1.17 & 2.18; 1.17 & 2.19; 1.17 & 2.20; 1.17 & 2.21; 1.17 & 2.22; 1.17 & 2.23; 1.17 & 2.24; 1.17 & 2.25; 1.17 & 2.28; 1.17 & 2.29; 1.17 & 2.30; 1.17 & 2.31; 1.17 & 2.32; 1.17 & 2.33; 1.17 & 2.34; 1.18 & 2.01; 1.18 & 2.02; 1.18 & 2.03; 1.18 & 2.04; 1.18 &2.05; 1.18 & 2.06; 1.18 & 2.07; 1.18 & 2.08; 1.18 & 2.09; 1.18 & 210; 1.18 & 211; 1.18 & 2.12; 1.18 & 2.13; 1.18 & 2.14; 1.18 & 2.15; 1.18 & 2.16; 1.18 & 2.17; 1.18 & 2.18:1.18 & 2.19; 1.18 & 2.20; 1.18 & 2.21; 1.18 & 2.22; 1.18 & 2.23; 1.18 & 2.24; 1.18 & 2.25; 1.18 & 2.28; 1.18 & 2.29; 1.18 & 2.30; 1.18 & 2.31; 1.18 & 2.32;1.18 & 2.33; 1.18 & 2.34; 1.19 & 2.01; 1.19 & 2.02; 1.19 & 2.03; 1.19 & 2.04; 1.19 & 2.05; 1.19 & 2.06; 1.19 & 2.07; 1.19 & 2.08; 1.19 & 2.09; 1.19 & 210; 1.19 & 211; 1.19 & 2.12; 1.19 & 2.13; 1.19 & 2.14; 1.19 & 2.15; 1.19 & 2.16; 1.19 & 2.17; 1.19 & 2.18; 1.19 & 2.19; 1.19 & 2.20; 1.19 &2.21; 1.19 & 2.22; 1.19 & 2.23; 1.19 & 2.24; 1.19 & 2.25; 1.19 & 2.28; 1.19 & 2.29; 1.19 & 2.30; 1.19 & 2.31; 1.19 & 2.32; 1.19 & 2.33; 1.19 & 2.34; 1.20 & 2.01; 1.20 & 2.02; 1.20 & 2.03; 1.20 & 2.04; 1.20 & 2.05; 1.20 & 2.06; 1.20 & 2.07; 1.20 & 2.08; 1.20 & 2.09; 1.20 & 210; 1.20 & 211; 1.20 & 2.12; 1.20 & 2.13; 1.20 & 2.14; 1.20 & 2.15; 120 & 2.16; 1.20 & 2.17; 1.20 & 2.18; 1.20 & 2.19; 1.20 & 2.20; 1.20 & 2.21; 1.20 & 2.22; 1.20 & 2.23; 1.20 & 2.24; 1.20 & 2.25; 1.20 & 2.28; 1.20 & 2.29; 1.20 & 2.30; 1.20 & 2.31; 1.20 & 2.32; 1.20 & 2.33; 1.20 & 2.34; 1.21 & 2.01; 1.21 & 2.02; 1.21 & 2.03; 1.21 & 2.04; 1.21 & 2.05; 1.21 & 2.06; 1.21 & 2.07; 1.21 & 2.08; 1.21 & 2.09; 1.21 & 210; 1.21 & 211; 1.21 & 2.12; 1.21 & 2.13; 1.21 & 2.14; 1.21 & 2.15; 1.21 & 2.16; 1.21 & 2.17; 1.21 & 2.18; 1.21 & 2.19; 1.21 & 2.20; 1.21 & 2.21; 1.21 & 2.22; 1.21 & 2.23; 1.21 & 2.24; 1.21 & 2.25; 1.21 & 2.28; 1.21 & 2.29; 1.21 & 2.30; 1.21 & 2.31; 1.21 & 2.32; 1.21 & 2.33; and 1,21 & 2.34.

The following examples and patent claims illustrate the present invention, but in no way limit its scope. The temperatures are given in degrees Celsius.

In the following formulation examples, the term the compound of formula (I) represents (E)-N-(6-chloro-3-pyridylmethyl)-N-ethyl-N'-methyl-2-nitrovinylidenediamine or (*R,S*)-1-[2,5-dichloro-4-(1,1,2,2,3,3-hexafluoropropoxy)-

phenyl]-3-(2,6-difluorobenzoyl)urea.

Example 1: Tablets containing 25 mg of the compound of formula (I) or (II) or of both active substances may be produced as follows:

| Constituents (for 1000 tablets) | |
|---|---|
| active ingredient | 25.0 g |
| lactose | 100.7 g |
| wheat starch | 7.5 g |
| polyethylene glycol 6000 | 5.0 g |
| talc | 5.0 g |
| magnesium stearate | 1.8 g |
| demineralised water | q.s. |

Preparation: First of all, all solid ingredients are forced through a sieve of 0.6 mm mesh size. Then, the active ingredient, the lactose, the talc and half of the starch are admixed. The other half of the starch is suspended in 40 ml of water and this suspension is added to a boiling solution of the polyethylene glycol in 100 ml of water. The starch paste obtained is added to the principle amount and the mixture granulated, if necessary adding water. The granulate is dried over night at 35°, forced through a sieve of 1.2 mm mesh size, mixed with the magnesium stearate and pressed into biconcave tablets of ca. 6 mm diameter.

Example 2: Tablets containing 0.02 g of the compound of formula (I) or (II) or of both active substances may be produced as follows:

| Constituents (for 10,000 tablets) | |
|---|---|
| active ingredient | 200.00 g |
| lactose | 290.80 g |
| potato starch | 274.70 g |
| stearic acid | 10.00 g |
| talc | 200.00 g |
| magnesium stearate | 2.50 g |
| colloidal silicon dioxide | 32.00 |
| ethanol | q.s. |

A mixture of a compound of formula (I), the lactose and 194.70 g of potato starch is moistened with an ethanolic solution of the stearic acid and granulated through a sieve. After drying, the remaining potato starch, the talc, the magnesium stearate and the colloidal silicon dioxide are mixed in, and the mixture is pressed into tablets each of 0.1 g weight, which may be provided with dividing notches for finer adjustment of the dosage.

Example 3: Capsules containing 0.025 g of a compound of formula (I) or (II) or of both active substances may be produced as follows:

| Constituents (for 1000 capsules) | |
|---|---|
| active ingredient | 25.00 g |
| lactose | 249.80 g |
| gelatin | 2.00 g |
| corn starch | 10.00 g |
| talc | 15.00 g |
| water | q.s. |

The active ingredient(s) is/are mixed with the lactose, the mixture is moistened evenly with an aqueous solution of the gelatin and granulated through a sieve having a mesh size of 1.2-1.5 mm. The granulate is mixed with the dried corn starch and the talc, and 300 mg portions thereof are filled into hard gelatin capsules (size 1).

Example 4: Premix (food additive)

| | |
|---|---|
| 0.25 | parts by weight active ingredient and |
| 4.75 | parts by weight secondary calcium phosphate, alumina, aerosil, carbonate or lime are mixed to homogeneity with |
| 95 | parts by weight of an animal feed. |

Example 5: Premix (food additive)

| | |
|---|---|
| 0.40 | parts by weight active ingredient and |
| 5.00 | parts by weight of aerosil/lime (1:1 ) are mixed to homogeneity with |
| 94.6 | parts by weight of a commercial dry food. |

Example 6: Emulsifier concentrate

| | |
|---|---|
| 20 | parts by weight active ingredient are mixed with |
| 20 | parts by weight of an emulsifier, e.g. a mixture of alkylaryl polyglycol ether with alkylaryl polysulphonates, and with |
| 60 | parts by weight of a solvent until the solution has become completely homogenised. By diluting with water, emulsions of a desired concentration are obtained. |

Example 7: Solutions (e.g. for usage as a drink additive)

| | |
|---|---|
| 15 | percent by weight of active ingredient, |
| 10 | percent by weight of the active ingredient in diethylene glycol monoethylether, |
| 10 | percent by weight in polyethylene glycol 300, and |
| 5 | percent by weight in glycerol. |

Example 8: Soluble powder

| | |
|---|---|
| 25 | parts by weight of active ingredient |
| 1 | part by weight of sodium lauryl sulphate, |
| 3 | parts by weight of colloidal silica gel, and |
| 71 | parts by weight of urea. |

The constituents are mixed and ground together until homogeneous.

Further biologically active substances or additives, which are neutral towards the active ingredients and do not have a harmful effect on the host animal to be treated, as well as mineral salts or vitamins, may be added to the compositions described.

Further preparations with active substances of formula (I) and/or (II) may also be prepared analogously to the above-described formulations of examples 1 to 8.

Biological example

Example B1: Comparison between the activity of lufenuron alone and the combination of nitenpyran and lufenuron aqainst dog fleas in a contaminated environment corresponding to actual conditions.

| | |
|---|---|
| Location: | Parasitology and Dermatology department of Toulouse Veterinary School, Toulouse, France. |
| Type of study: | Activity against fleas |
| Period: | Spring to autumn 1997 |
| Animals being tested: | 20 dogs, healthy Beagles of the same breed |
| Parasite: | Cat flea *Ctenocephalides felis* |
| Procedure: | Placebo-controlled; blind test; simulation of the natural environment (domestic) |
| Treatment method: | Determination of efficacy in a contaminated environment following oral administration of tablets |
| Test substances: | Nitenpyram as palatable, round, biconvex and scented tablets of 100 mg (11.4 mg active substance) for dogs of 1.0 to 11.0 kg and 500 mg (57 mg active substance) for dogs of 11.1 to 57 kg<br>Lufenuron was administered in the form of commercially available PROGRAM® M tablets containing 204.9 mg lufenuron for dogs of 7 to 29 kg body weight |
| Placebo: | Palatable, round, biconvex and scented 100 and 500 mg tablets as above, but without the active ingredient |

In the study, conditions existing in the household were simulated. To this end, 4 isolated and clinically clean rooms were used. They contained a sleeping area laid out with straw and a small run to the outside. The rooms were subjected to natural light conditions. 5 healthy dogs were placed in each room and each were infected three times a week with 50 freshly hatched fleas, until the dogs had a constant flea population. Subsequently, each group of dogs underwent different treatment.
Control group 1 was given no active ingredient, but only a placebo and served as the reference group. Group 2 was given lufenuron in a monthly dosage in accordance with the instructions on the packet and once a week was given a placebo. Group 3 was given lufenuron in a monthly dosage in accordance with the instructions on the packet, and once a week was additionally given nitenpyram for a period of 6 weeks. Group 4 was given lufenuron in a monthly dosage in accordance with the instructions on the packet, and twice a week was additionally given nitenpyram for a period of 6 weeks.
This was a blind test, in which treatment and evaluation were carried out by different people who did not know the test protocol.
In all four groups, the flea population was determined by counting them twice weekly before the first treatment and once weekly during treatment.
The activity of the test substances was determined under simulated household conditions in accordance with the following formula:

$$\text{acivity in \%} = \frac{(\text{number of fleas in the control group}) - (\text{number of fleas in the treated group})}{(\text{numbers of fleas in the control group})} \times 100$$

After the first administration of nitenpyram, all the fleas except one died within 16-24 days, but there were clear differences during the whole treatment. The results are summarised in Table 1.

Table 1:

| Activity (% relative to control) of groups 1 - 4 after the first treatment | | | | |
|---|---|---|---|---|
| **Group/days** | **0 - 28** | **29 - 56** | **57 - 84** | **85-112** |
| Group 1<br>control only placebo | 0 | 0 | 0 | 0 |

Table 1: (continued)

| Activity (% relative to control) of groups 1 - 4 after the first treatment | | | | |
|---|---|---|---|---|
| **Group/days** | **0 - 28** | **29 - 56** | **57 - 84** | **85-112** |
| Group 2<br>only lufenuron | 25.4 | 78.6 | 72.1 | 94.3 |
| Group 3<br>lufenuron + once weekly nitenpyram | 94.2 | 99.9 | 99.9 | 100 |
| Group 4<br>lufenuron + twice weekly nitenpyram | 84.3 | 99.1 | 99.8 | 99.8 |

As could be seen, the activity of group 2, which had been treated solely with lufenuron, was very low up to day 28 in comparison with groups 3 and 4, and only achieved a satisfactory level for practical usage after ca. 84 days. In contrast, the activity in groups 3 and 4 was considerably higher from the outset and reached full effect after at latest 28 days. Groups 3 and 4 were not significantly different from one another, i.e. no substantial differences were established in the once or twice weekly administration of nitenpyram.

## Claims

**1.** Veterinary composition for the control of fleas comprising an amount that is effective against fleas of a combination of a compound of formula (I)

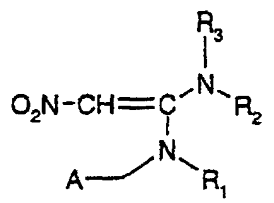

(I)

wherein

$R_1$ is hydrogen, $C_1$-$C_6$alkyl or $C_3$-$C_7$-cycloalkyl;
$R_2$ is hydrogen, $C_1$-$C_6$alkyl or $C_3$-$C_7$-cycloalkyl;
$R_3$ is hydrogen or $C_1$-$C_6$alkyl; and
A is heterocyclyl which is unsubstituted or substituted once or repeatedly by identical or different halogen atoms;

and a compound of formula (II)

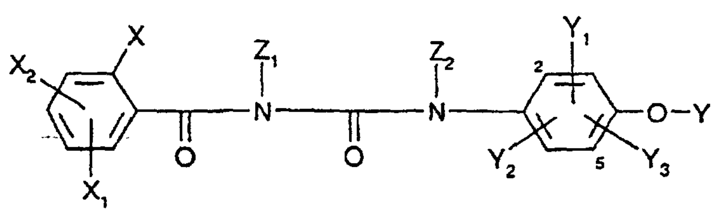

(II)

wherein

X is halogen;
$X_1$ is hydrogen or halogen;
$X_2$ is hydrogen or halogen;
Y is partially or completely halogenated $C_1$-$C_6$-alkyl, or completely halogenated $C_1$-$C_6$-alkyl which is interrupted

by one oxygen atom, or partially or completely halogenated $C_2$-$C_6$-alkenyl;
$Y_1$ is hydrogen or halogen;
$Y_2$ is hydrogen or halogen;
$Y_3$ is hydrogen or halogen;
$Z_1$ is hydrogen or $C_1$-$C_3$-alkyl; and
$Z_2$ is hydrogen or $C_1$-$C_3$-alkyl; and

and a physiologically acceptable formulation excipient.

**2.** Use of a compound of formula (I)

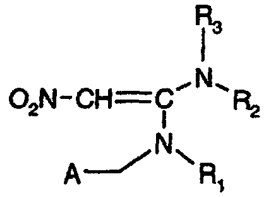

(I)

wherein

$R_1$ is hydrogen, $C_1$-$C_6$alkyl or $C_3$-$C_7$-cycloalkyl;
$R_2$ is hydrogen, $C_1$-$C_6$alkyl or $C_3$-$C_7$-cycloalkyl;
$R_3$ is hydrogen or $C_1$-$C_6$alkyl; and
A is heterocyclyl which is unsubstituted or substituted once or repeatedly by identical or different halogen atoms;

in combination with a compound of formula (II)

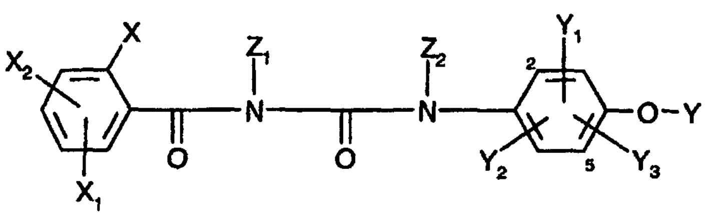

(II)

wherein

X is halogen;
$X_1$ is hydrogen or halogen;
$X_2$ is hydrogen or halogen;
Y is partially or completely halogenated $C_1$-$C_6$-alkyl, or completely halogenated $C_1$-$C_6$-alkyl, which is interrupted by one oxygen atom, or partially or completely halogenated $C_2$-$C_6$-alkenyl;
$Y_1$ is hydrogen or halogen;
$Y_2$ is hydrogen or halogen;
$Y_3$ is hydrogen or halogen;
$Z_1$ is hydrogen or $C_1$-$C_3$-alkyl; and
$Z_2$ is hydrogen or $C_1$-$C_3$-alkyl, for the manufacture of a veterinary medicament for the control of fleas on domestic animals.

**3.** Use according to claim 2, whereby the veterinary medicament is an oral administration form.

**4.** Use according to claim 2, whereby the veterinary medicament consists of separate dosage forms of the compound of formula (I) and that of formula (II) but wherein both dosage forms are administered in parallel.

**5.** Use according to claim 2, whereby the veterinary medicament consists of separate dosage forms of the compound

of formula (I) and that of formula (II) and wherein the dosage form of the compound of formula (I) is applied first and the treatment with the compound of formula (II) is carried out subsequently.

## Patentansprüche

1. Veterinärzusammensetzung für die Bekämpfung von Flöhen, umfassend eine gegen Flöhe wirksame Menge einer Kombination einer Verbindung der Formel (I)

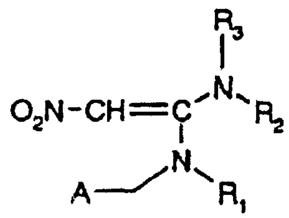

(I)

worin $R_1$ Wasserstoff, $C_1$-$C_6$-Alkyl oder $C_3$-$C_7$-Cycloalkyl darstellt;
$R_2$ Wasserstoff, $C_1$-$C_6$-Alkyl oder $C_3$-$C_7$-Cycloalkyl darstellt;
$R_3$ Wasserstoff oder $C_1$-$C_6$-Alkyl darstellt und
A Heterocyclyl darstellt, das unsubstituiert oder einmal oder wiederholt mit gleichen oder verschiedenen Halogenatomen substituiert ist;
und einer Verbindung der Formel (II)

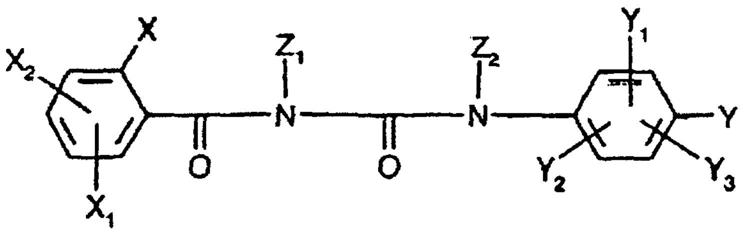

(II)

worin
X Halogen darstellt;
$X_1$ Wasserstoff oder Halogen darstellt;
$X_2$ Wasserstoff oder Halogen darstellt;
Y teilweise oder vollständig halogeniertes $C_1$-$C_6$-Alkyl oder vollständig halogeniertes $C_1$-$C_6$-Alkyl, das durch ein Sauerstoffatom unterbrochen ist, oder teilweise oder vollständig halogeniertes $C_2$-$C_6$-Alkenyl darstellt;
$Y_1$ Wasserstoff oder Halogen darstellt;
$Y_2$ Wasserstoff oder Halogen darstellt;
$Y_3$ Wasserstoff oder Halogen darstellt;
$Z_1$ Wasserstoff oder $C_1$-$C_3$-Alkyl darstellt und
$Z_2$ Wasserstoff oder $C_1$-$C_3$-Alkyl darstellt und einen physiologisch verträglichen Formulierungsexzipienten.

2. Verwendung einer Verbindung der Formel (I)

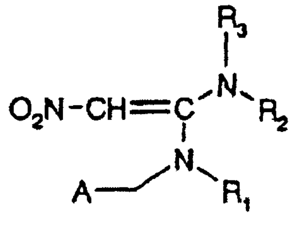

(I)

worin $R_1$ Wasserstoff, $C_1$-$C_6$-Alkyl oder $C_3$-$C_7$-Cycloalkyl darstellt;
$R_2$ Wasserstoff, $C_1$-$C_6$-Alkyl oder $C_3$-$C_7$-Cycloalkyl darstellt;
$R_3$ Wasserstoff oder $C_1$-$C_6$-Alkyl darstellt und
A Heterocyclyl darstellt, das unsubstituiert oder einmal oder wiederholt mit gleichen oder verschiedenen Halogenatomen substituiert ist;
in Kombination mit einer Verbindung der Formel (II)

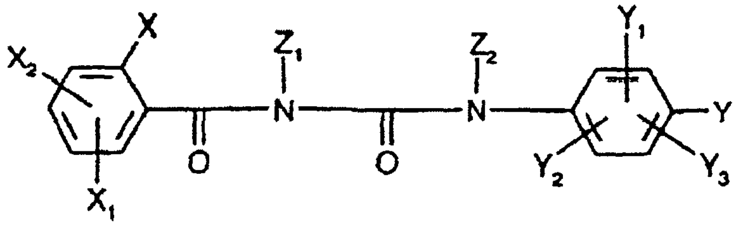 (II)

worin
X Halogen darstellt;
$X_1$ Wasserstoff oder Halogen darstellt,
$X_2$ Wasserstoff oder Halogen darstellt;
Y teilweise oder vollständig halogeniertes $C_1$-$C_6$-Alkyl oder vollständig halogeniertes $C_1$-$C_6$-Alkyl, das durch ein Sauerstoffatom unterbrochen ist, oder teilweise oder vollständig halogeniertes $C_2$-$C_6$-Alkenyl darstellt;
$Y_1$ Wasserstoff oder Halogen darstellt;
$Y_2$ Wasserstoff oder Halogen darstellt;
$Y_3$ Wasserstoff oder Halogen darstellt;
$Z_1$ Wasserstoff oder $C_1$-$C_3$-Alkyl darstellt und
$Z_2$ Wasserstoff oder $C_1$-$C_3$-Alkyl darstellt; zur Herstellung eines Veterinärarzneimittels für die Bekämpfung von Flöhen auf Haustieren.

3. Verwendung nach Anspruch 2, wobei das Veterinärarzneimittel eine orale Verabreichungsform ist.

4. Verwendung nach Anspruch 2, wobei das Veterinärarzneimittel aus getrennten Dosierungsformen der Verbindung der Formel (I) und jener von Formel (II) besteht, jedoch worin beide Dosierungsformen parallel verabreicht werden.

5. Verwendung nach Anspruch 2, wobei das Veterinärarzneimittel aus getrennten Dosierungsformen der Verbindung der Formel (I) und jener der Formel (II) besteht und wobei die Dosierungsform der Verbindung der Formel (I) zuerst verabreicht wird und die Behandlung mit der Verbindung der Formel (II) anschließend ausgeführt wird.

## Revendications

1. Composition vétérinaire pour lutter contre les puces, comprenant une quantité, qui est efficace contre les puces, d'une combinaison d'un composé de formule (I) :

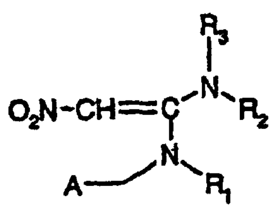 (I)

dans laquelle :

$R_1$ est l'hydrogène, un groupe alkyle en $C_1$-$C_6$ ou cycloalkyle en $C_3$-$C_7$;
$R_2$ est l'hydrogène, un groupe alkyle en $C_1$-$C_6$ ou cycloalkyle en $C_3$-$C_7$;

$R_3$ est l'hydrogène ou un groupe alkyle en $C_1$-$C_6$; et
A est un groupe hétérocyclyle qui est non substitué ou substitué une fois ou de manière répétée par des atomes d'halogène identiques ou différents;

et d'un composé de formule (II):

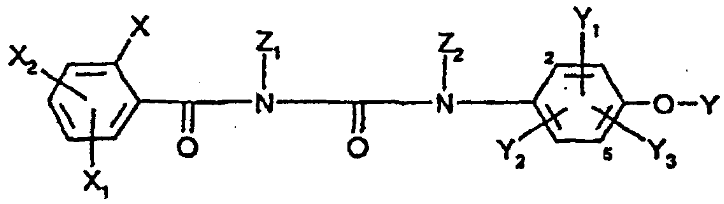
(II)

dans laquelle :

X est un halogène;
$X_1$ est l'hydrogène ou un halogène;
$X_2$ est l'hydrogène ou un halogène;
Y est un groupe alkyle en $C_1$-$C_6$ partiellement ou complètement halogéné ou un groupe alkyle en $C_1$-$C_6$ complètement halogéné qui est interrompu par un atome d'oxygène, ou un groupe alcényle en $C_2$-$C_6$ partiellement ou complètement halogéné;
$Y_1$ est l'hydrogène ou un halogène;
$Y_2$ est l'hydrogène ou un halogène;
$Y_3$ est l'hydrogène ou un halogène;
$Z_1$ est l'hydrogène ou un groupe alkyle en $C_1$-$C_3$; et
$Z_2$ est l'hydrogène ou un groupe alkyle en $C_1$-$C_3$; et

un excipient de formulation physiologiquement acceptable.

**2.** Utilisation d'un composé de formule (I):

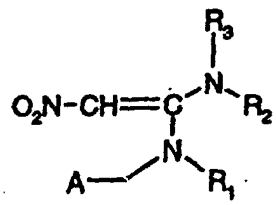
(I)

dans laquelle :

$R_1$ est l'hydrogène, un groupe alkyle en $C_1$-$C_6$ ou cycloalkyle en $C_3$-$C_7$;
$R_2$ est l'hydrogène, un groupe alkyle en $C_1$-$C_6$ ou cycloalkyle en $C_3$-$C_7$;
$R_3$ est l'hydrogène ou un groupe alkyle en $C_1$-$C_6$; et
A est un groupe hétérocyclyle qui est non substitué ou substitué une fois ou de manière répétée par des atomes d'halogène identiques ou différents;

en combinaison avec un composé de formule (II):

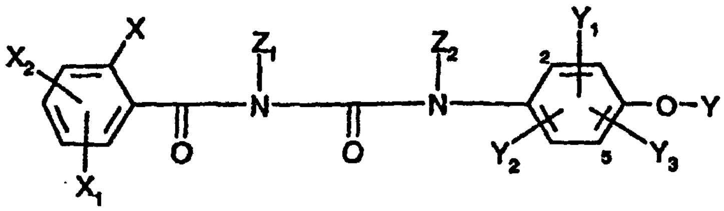

(II)

dans laquelle :

X est un halogène;
$X_1$ est l'hydrogène ou un halogène;
$X_2$ est l'hydrogène ou un halogène;
Y est un groupe alkyle en $C_1$-$C_6$ partiellement ou complètement halogéné ou un groupe alkyle en $C_1$-$C_6$ complètement halogéné qui est interrompu par un atome d'oxygène ou un groupe alcényle en $C_2$-$C_6$ partiellement ou complètement halogène,
$Y_1$ est l'hydrogène ou un halogène;
$Y_2$ est l'hydrogène ou un halogène;
$Y_3$ est l'hydrogène ou un halogène;
$Z_1$ est l'hydrogène ou un groupe alkyle en $C_1$-$C_3$; et
$Z_2$ est l'hydrogène ou un groupe alkyle en $C_1$-$C_3$, pour la fabrication

d'un médicament vétérinaire pour lutter contre les puces sur des animaux domestiques.

3. Utilisation selon la revendication 2, dans laquelle le médicament vétérinaire est une formulation à administration orale.

4. Utilisation selon la revendication 2, dans laquelle le médicament vétérinaire consiste en formes posologiques séparées du composé de formule (I) et de celui de formule (II), mais dans laquelle les deux formes posologiques sont administrées en parallèle.

5. Utilisation selon la revendication 2, dans laquelle le médicament vétérinaire consiste en formes posologiques séparées du composé de formule (I) et de celui de formule (II) et dans laquelle la forme posologique du composé de formule (I) est appliquée en premier lieu et le traitement avec le composé de formule (II) est effectué ultérieurement.